# EUROPEAN PATENT APPLICATION

(11) **EP 2 524 696 A1**
(43) Date of publication of application: **21.11.2012**
(21) Application number: 11166772.1
(22) Date of filing: 19.05.2011
(51) Int. Cl.: A61K 36/185, A61K 36/53

(54) **Alcohol-free pharmaceutical composition comprising thyme and primula root liquid extracts**

(71) Applicant: A. Nattermann & Cie. GmbH, 50829 Köln (DE)
(72) Inventor: Holzstein, Werner, 65926, FRANKFURT AM MAIN (DE); Weissenfeld, Jörn, 65926, FRANKFURT AM MAIN (DE); Hoffmann, Verena, 65926, FRANKFURT AM MAIN (DE)
(74) Representative: Weber, Mathieu

(57) **Abstract**

The present invention relates to an alcohol-free liquid pharmaceutical composition comprising thyme liquid extract and primula root liquid extract and optionally a non-alcoholic solvent and excipients, preferably chosen from sucrose, invert sugar, water and mixtures thereof. The alcohol-free liquid pharmaceutical composition of present invention intends preferably to have the same herbal fingerprint as Bronchicum Elixir alcohol-containing composition.

The invention also discloses methods for preparing said alcohol-free liquid pharmaceutical composition.

The invention relates also to a thyme and primula root liquid extract that may be obtained according to one of the methods described and to the use of the said thyme and primula root liquid extract for the preparation of a pharmaceutical composition.

## Description

The present invention relates to alcohol-free liquid pharmaceutical compositions comprising thyme liquid extract and primula root liquid extract and methods for preparing these compositions. The alcohol-free liquid pharmaceutical compositions of the invention have the same herbal fingerprint as the corresponding pharmaceutical composition prepared with alcohol-containing thyme and primula root liquid extracts.

Liquid plant extracts are often introduced in pharmaceutical products and are obtained by extraction of a plant with a suitable solvent such as a mixture of water and alcohol. Sometimes, alcohol has been used also as a diluent to liquid herbal preparations. The resulting extracts could therefore comprise a significant amount of alcohol, such as ethanol, which will end up in the finished pharmaceutical product..

One major drawback of herbal medicinal products for oral administration containing high level of alcohol is that they are not suitable for any patient population, and for example cannot be administered to patients having liver diseases or patients with religious beliefs forbidding the consumption of alcohol. Specifically, a systemic ethanol exposure by products where ethanol is present in substantial amounts, e.g., ethanol concentration in excess of 60% (v/v) in the finished oral liquid product intended for the paediatric population may have toxicological concerns with respect to both short-term and prolonged use. Furthermore, for paediatric use, concomitant use of other medicinal product that contains ethanol should be avoided; the dose interval should be kept as long as possible with at least 4 hours to avoid accumulation; and the whole treatment period should be as short as possible. Lastly, alcohol such as ethanol, may enhance the absorption and pharmacological effect of some drugs, and affect the elimination of others.

Therefore, appropriateness and safety of alternatives to alcohol such as ethanol should be considered and efforts should be made to have replaced ethanol in herbal liquid preparations. Consideration should be given to herbal medicinal product containing a lowest level of alcohols or even better no alcohol.

Bronchicum Elixir is a traditional herbal product formulated with alcohol-containing thyme and primula root liquid extracts, and containing a final amount of ethanol between about 4 and 5% (v/v). There is a need for removing ethanol form this Elixir while not modifying the herbal fingerprint of the original pharmaceutical formulation.

A well known method for removing alcohol from liquid formulations is distillation. However, other volatile compounds contained in some liquid plant extracts can be removed with the alcohols upon distillation process, therefore modifying the herbal fingerprint of the resulting alcohol-free formulation compared to the fingerprint of the original formulation.

Document EP-A-0589921 discloses a method for preparing an alcohol-free plant extract comprising the distillation of a mixture of water and alcohol comprising a plant extract in a first step, then a separation of the distilled alcohol and volatile compounds with a suitable extracting agent and finally, the admixture of the distillate residue with the recovered volatile compounds. However, the choice of the appropriate extracting agent mainly depends on volatile compounds present in the alcohol-containing plant extract. EP-A-0589921 only illustrates isopropyl myristate as extracting agent for the separation of vitex agnus castus extract from ethanol, and gives no indication on how to remove alcohol from other plant liquid extract, such as thyme or primula root liquid extract.

Document EP-A-0708650 discloses the preparation of alcohol-free plant extracts by extracting the plant, for example thyme, with a mixture of water and a non-ionic surfactant instead of the standard mixture of water and alcohol. However, when preparing plant extract-containing liquid formulations, pharmaceutical companies usually purchase a ready-to-use liquid plant extract to a supplier and do not proceed with the extraction of the plant itself. The process disclosed in EP-A-0708650 teaches how to prepare plant extracts without using alcohol does not disclose how to remove alcohol from alcohol-containing plant extracts.

There remains therefore a need for an alcohol-free liquid formulation comprising thyme liquid extract and primula root liquid extract having the same herbal fingerprint as the alcohol-containing corresponding composition, as well as methods for preparing such formulations.

It is therefore a first object of the present invention to provide an alcohol-free liquid pharmaceutical composition comprising thyme liquid extract and primula root liquid extract and optionally a non-alcoholic solvent and excipients, preferably chosen from sucrose, invert sugar, water and mixtures thereof.

In another aspect, the present invention relates to a method for preparing said alcohol-free pharmaceutical composition comprising the step of:
- removing alcohol from alcohol-containing thyme and primula root liquid extracts by either nanofiltration or reversed osmosis, and,
- optionally, adding a non-alcoholic solvent and excipients, preferably chosen from sucrose, invert sugar, water and mixtures thereof.

In yet another aspect, the invention is directed to another method for preparing said alcohol-free pharmaceutical composition comprising the steps of:
i. Spraying a granulating solution comprising an alcohol-containing thyme liquid extract and alcohol-containing primula root liquid extract on a sugar matrix to obtain thyme- and primula root-extract containing granules, and
ii. Dissolving the obtained granules into a non-alcoholic solvent and excipients.

In another object, the invention relates to a thyme and primula root liquid extract that may be obtained according to one of the methods described above.

In another objet, the invention is directed to the use of a thyme and primula root liquid extract as obtained above for the preparation of a pharmaceutical composition.

The alcohol-free liquid pharmaceutical composition of present invention intends preferably to have the same herbal fingerprint as Bronchicum Elixir alcohol-containing composition.

The Composition of the prior art Bronchicum Elixir formulation is the following:

| **Raw Materials** | **Function** | **Formula per 100 g** |
|---|---|---|
| Thyme liquid extract (1 : 2-2.5) | Drug substance | 5.00 |
| Primula root liquid extract (1 : 2-2.5) | Drug substance | 2.50 |
| Sodium benzoate | Preservative | 0.18 |
| Mixture of sucrose and invert syrup | Flavour, Solvent | 85.00 |
| Purified water | Solvent | 7.32 |

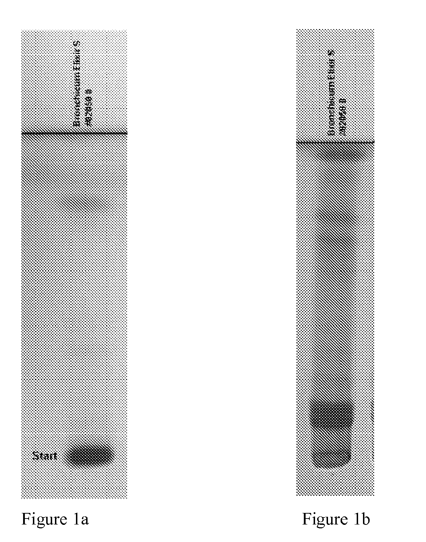

The TLC fingerprint of this composition is illustrated in figure 1a-b.

The TLC conditions used for the fingerprint of Thyme liquid extract illustrated in figure 1a are the following:

| | |
|---|---|
| Method: | One-dimensional development without chamber saturation |
| Plate: | Silica gel plate 60 F254; 0.25mm layer; 20x20cm |
| Application: | 20µl in 2cm strips |
| Mobile phase: | Dichloromethane |
| Flow: | 10cm, twice |
| Detection: | Spraying the plate after development with anisaldehyde spray reagent*, then heated to 100°C *Anisaldehyde/acetic acid/ methanol/ sulfuric acid 96% 0.5/10/85/5 v/v/v/v. |

The TLC conditions used for the fingerprint of Primula root liquid extract illustrated in figure 1b are the following:

| | |
|---|---|
| Method: | One-dimensional development without chamber saturation |
| Plate: | Silica gel plate Si 60 F254; 0.25mm layer; 20x20cm |
| Application: | 20µl in 2cm strips |
| Mobile phase: | Chloroform/ methanol/ water 60/30/5 v/v/v |
| Flow: | 10cm |
| Detection: | Drying the plate after development at 70°C; spraying the plate with vanillin in ethanol (1g in 100ml) and then with sulphuric acid 96% in ethanol (5g in 100ml), then heated to 100°C. |

The specific embodiment wherein alcohol-free liquid pharmaceutical composition of the invention has the same herbal fingerprint as alcohol-containing Bronchicum Elixir composition is intended to mean, in the context of the present invention, that the chromatographic (either liquid HPLC, gaseous GC or thin layer TLC) fingerprints of the alcohol-free formulation of the invention and of the alcohol-containing Bronchicum Elixir formulation show the presence of the same compounds (i.e. show the same picks in HPLC or tracks in GC and TLC). Their HPLC fingerprints for example can almost be superimposed.

In the same manner, by the expression "having the same herbal fingerprint as the alcohol-containing corresponding herbal extract", it is intended to mean, in the context of the present invention, that the chromatographic (either liquid HPLC or gaseous GC or thin layer TLC) fingerprints of the alcohol-free herbal extract and of its corresponding alcohol-containing extract show the presence of the same compounds (i.e. show the same picks in HPLC or tracks in GC or TLC). Their HPLC fingerprints for example can almost be superimposed.

### Figures

**Figure 1**a-b illustrates the herbal fingerprint of Bronchicum Elixir alcohol-containing composition:
   Figure 1a illustrates, the TLC phenolic compound fingerprint of Bronchicum Elixir, and, figure 1b illustrates the TLC saponins fingerprint of Bronchicum Elixir.
**Figure 2** illustrates the HPLC fingerprint of primulic acid in the original primula root alcohol-containing extract and in the retentate (b) of membrane filtration corresponding to an alcohol-free primula root extract according to example 1.
**Figure 3** illustrates the GC fingerprint of thymol in the original alcohol-containing thyme extract and in the retentate (d) of membrane filtration corresponding to an alcohol-free thyme extract according to example 3.
**Figure 4** illustrates the HPLC thymol fingerprint of the original mixture of primula root and thyme alcohol-containing extracts and of the retentate (e) of membrane filtration corresponding to the alcohol-free thyme and primula root extracts according to example 5.
**Figure 5** illustrates the HPLC primulic acid fingerprint of the original mixture of primula root and thyme alcohol-containing extracts and of the retentate (e) of membrane filtration corresponding to the alcohol-free thyme and primula root extracts according to example 5.

### Alcohol-free pharmaceutical composition

The present invention is directed to a liquid pharmaceutical composition which is free of alcohol.

In the context of the present invention, the term "alcohol-free" is intending to designate pharmaceutical compositions containing less than about 5000 mg/kg (ppm) of alcohol, in particular less than about 5000 mg/kg (ppm) of (C₁-C₆-alkyl)-OH.

In a more specific embodiment, the term "alcohol-free" pharmaceutical composition is intending to designate, pharmaceutical compositions containing less than about 5000 ppm of ethanol.

### Thyme and Primula Root liquid extracts

The liquid pharmaceutical composition according to the present invention comprises alcohol-free thyme and primula root liquid extracts.

### Thyme liquid extract:

Thyme is a medicinal herb of the genus *Thymus.*

The alcohol-containing thyme liquid extract used for the preparation of the pharmaceutical composition of the invention comprises an initial amount of 30 to 37 % in volume of alcohol (ethanol), which is intended to be removed.

In addition to ethanol, the alcohol-containing thyme liquid extract further contains the phenolic compounds thymol and carvacrol.

The alcohol-containing thyme liquid extract used for the preparation of the pharmaceutical composition of the invention can for example be purchased from R&R Extrakte GmbH, Cologne Germany under the commercial reference #223094.

### Primula root liquid extract:

Primula is a genus of 400-500 species of low-growing herbs in the family Primulaceae.

The alcohol-containing primula root liquid extract used for the preparation of the pharmaceutical composition of the invention comprises an initial amount of 72 to 78.5 % in volume of alcohol (ethanol), which is intended to be removed.

In addition to ethanol, the alcohol-containing primula root liquid extract further contains saponins like primulic acid I and II.

The alcohol-containing primula root liquid extract used for the preparation of the pharmaceutical composition of the invention can for example be purchased from R&R Extrakte GmbH, Cologne Germany under the commercial reference #223072.

In a preferred embodiment, the alcohol-free formulation of the invention comprise a weight ratio of thyme liquid extract to primula root liquid extract of 2:1 to 5:1, and preferably is about 4:1.

The total amount of thyme and primula root liquid extracts in the alcohol-free formulation of the invention is ranging from 0.1 to 10% by weight, preferably from 1 to 7% by weight, more preferably from 2 to 6%, and even more preferably about 4% by weight, relative to the total weight of the formulation.

### Solubilizer

In a particular embodiment, the alcohol-free thyme liquid extract or the primula root liquid extract or the mixture thereof can contain a solubilizer, to prevent their precipitation.

Without willing to be bounded by any theory, it can be assumed that some non water-soluble compounds present in the herbal extracts could precipitate when the ethanol is removed or replaced by water.

Suitable solubilizers can be chosen from but are not limited to triglycerides, glycerol esters, and mixtures thereof such as, for example:
- Caprylocaproyl polyoxyl-8-glycerides commercially available under the name Labrasol from Gattefossé Inc.,
- Linoleoyl macrogol-6 glycerides commercially available under the name Labrafil M 2125 CS from Gattefossé Inc.,
- Polyglyceryl-3 dioleate commercially available under the name Plurol Oleique from Gattefossé Inc.,
- Medium-chain triglycerides (C8-C10 fatty acids) commercially available under the name Labrafac Lipohile WL 1349 from Gattefossé Inc.,
- Highly purified diethylene glycol monoethyl ether commercially available under the name Transcutol HP from Gattefossé Inc.,
- Propylene glycol dicaprylocaprate commercially available under the name Labrafac PG from Gattefossé Inc.,
- Propylene glycol monocaprylate commercially available under the names Capryol 90 or Capryol PGMC from Gattefossé Inc.,
- Caprylic/capric triglycerides (triglycerides based on saturated C8-C10 fatty acids) commercially available under the name Myrtitol 318 PH from Gattefossé Inc. or Miglyol 812 from Sasol Germany GmbH,
- PEG-40 Hydrogenated Castor Oil commercially available under the name Cremophor RH 40 by BASF.

In a preferred embodiment, the solubilizer used in the formulation of the invention is PEG-40 hydrogenated castor oil.

The solubilizer can be present in the alcohol-free extract in an amount ranging from 0.01% and 20%, preferably from 0.1 and 15%, and even more preferably from 0.5 and 10% by weight, relative to the total weight of the alcohol-free extract.

### Liquid pharmaceutical composition of the invention

Further to the mixture of alcohol-free extracts of Primula root and thyme described above, the pharmaceutical composition according to the invention can contain some non-alcoholic solvents and excipients to allow an acceptable oral administration.

### Non-alcoholic solvent and excipients:

The alcohol-free liquid pharmaceutical composition of the present invention can optionally comprise a non-alcoholic solvent and excipients, preferably suitable for oral administration.

In a preferred embodiment, the non-alcoholic solvent and excipients can be chosen from but are not limited to sucrose, invert sugar, glucose, dextrin, fructose, galactose, mannose, maltodextrin, maltose, lactose, mannitol, sorbitol, xylitol, macrogol, water and mixtures thereof.

### Water:

Water, preferably purified water, can also be a suitable non-alcoholic solvent according to the invention.

Water can be present in the pharmaceutical composition of the invention in an amount ranging from about 1 to 40% by weight, preferably 5 to 35 % by weight.

### Sucrose and invert syrup:

Sucrose is the organic compound commonly known as sugar or saccharose. Sucrose is a disaccharide derived from glucose and fructose having the molecular formula C₁₂H₂₂O₁₁:

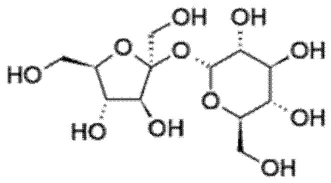

Invert syrup (or invert sugar syrup) is a mixture of glucose and fructose obtained by splitting sucrose into these two components through hydrolysis reaction.

In a preferred embodiment, the non-alcoholic pharmaceutical composition of the invention comprises a mixture of sucrose and invert syrup in a ratio ranging from 1:3 to 2:1, and preferably from 1:2 to 1:1.

Such mixture can for example be added to the pharmaceutical composition in an amount ranging from 60 to 95% by weight, relative to the total weigh of the pharmaceutical composition, preferably from 70 to 93% by weight, and more preferably 80 to 90% by weight.

### Granulating agent:

When the pharmaceutical composition of the invention is prepared by a method comprising a granulation step, a granulating agent can be used.

The granulating agent helps increasing the particle size of the granules, and consolidating the granules into a more uniform size distribution thus improving blending uniformity of herbal extracts.

The granulating agent can be selected from but is not limited to polymers such as methylcellulose, hydroxyethylcellulose, gelatin, carmellose sodium, hypromellose, polyvinylpyrrolidone (PVP), hydroxypropylcellulose, polyethylene glycol, starches such as cornstarch and pregelatinized starch, and modified starches.

Examples of granulating agents suitable in the context of the present invention include, but are not limited to, Povidone and PEG20000.

### Additives:

The pharmaceutical composition of the invention can further comprise any pharmaceutically acceptable additive, such as preservatives.

Suitable preservatives include, but are not limited to: sodium benzoate, methylparaben, propylparaben, benzoic acid, sorbic acid, chlorhexidine and the like.

In a particular embodiment, the pharmaceutical composition comprises sodium benzoate as suitable preservative.

### Method for preparing an alcohol-free pharmaceutical composition with nanofiltration or reverse osmosis

According to one particular aspect, the present invention relates to a method for preparing an alcohol-free pharmaceutical composition as previously disclosed comprising the steps of:
- removing alcohol from alcohol-containing thyme and primula root liquid extracts by either nanofiltration or reversed osmosis, and,
- optionally, adding a non-alcoholic solvent and excipients, preferably chosen from sucrose, invert sugar, water and mixtures thereof.

### Nanofiltration (NF):

The filtration process is the separation of solved, dispersed or emulsified molecules of different weight (size, shape or charge) by a molecule sieve (membrane). The choice of the membrane depends on the size of the molecule to be separated.

Nanofiltration (NF) is a cross-flow filtration technology. The nominal pore size of the membrane is typically about 1 nanometer.

Nanofiltration membranes are typically rated by molecular weight cut-off (MWCO) which is typically less than 1000 atomic mass units (daltons). The transmembrane pressure (pressure drop across the membrane) required for nanofiltration can be up to 40 bars.

Membranes suitable for alcohol removal from alcohol-containing thyme and primula root liquid extracts by nanofiltration preferably have a MWCO from 100 to 400 Daltons, and more preferably from 200 to 300 Daltons, roughly corresponding to a pore size ranging from 10⁻³ and 10⁻² µm.

Membranes suitable for alcohol removal from alcohol-containing thyme and primula root liquid extracts by nanofiltration can for example be purchased from Koch Membrane Systems under the commercial references SelRO® MPF34, SelRO® MPF44, TFC® SR3 and SR100, and preferably SelRO® MPF34 membrane is used.

In a specific embodiment, to prevent evaporation of highly volatile compounds such as thymol during the alcohol-removal step, and therefore to increase their recovery rate in the alcohol-free herbal extract, filtration unit parameters can be suitably selected.

In a preferred embodiment of the present invention, a Spirapro® filter unit available from Koch Membrane Systems can be used.

### Reverse osmosis (RO):

Reverse osmosis (RO) is a filtration method that removes many types of molecules and ions from solutions by applying pressure to the solution when it is on one side of a selective membrane.

In the normal osmosis process, the solvent naturally moves from an area of low solute concentration, through a membrane, to an area of high solute concentration. The movement of a pure solvent to equalize solute concentrations on each side of a membrane generates a pressure called "osmotic pressure." Applying an external pressure to reverse the natural flow of pure solvent is thus reverse osmosis.

The result is that the solute is retained on the pressurized side of the membrane and the pure solvent is allowed to pass to the other side. To be "selective," this membrane should not allow large molecules or ions through the pores, but should allow smaller components of the solution (such as the ethanol) to pass freely.

The membranes used for reverse osmosis have a dense barrier layer in the polymer matrix where most separation occurs. In most cases, the membrane is designed to allow only solvents such as water and ethanol to pass through this dense layer, while preventing the passage of solutes. This process requires that a high pressure be exerted on the high concentration side of the membrane, up to 100 bar.

Membranes suitable for alcohol removal from alcohol-containing thyme and primula root liquid extracts by reverse osmosis can for example be purchased from Koch Membrane Systems under the commercial references TFC®HR, TFC®XR, TFC®HF, TFC®SW and TFC®ULP by, and preferably TFC®HR membrane is used.

The step of removing alcohol from alcohol-containing thyme and primula root liquid extracts can be performed by either:
i. Removing separately the alcohol from the alcohol-containing thyme liquid extract, and from the alcohol-containing primula root liquid extract, optionally in presence of a solubilizer, by nanofiltration or reversed osmosis, or
ii. Admixing an alcohol-containing thyme liquid extract and an alcohol-containing primula root liquid extract, optionally in presence of a solubilizer, and Removing the alcohol from the alcohol-containing mixture of thyme and primula root liquid extracts by nanofiltration or reversed osmosis.

When the removal of alcohol is operated by first admixing the alcohol-containing thyme liquid extract and the alcohol-containing primula root liquid extract, precipitation of non water-soluble compounds can occur. A solubilizer can therefore be added to the mixture to prevent precipitation of compounds in the frame of the filtration unit.

In addition, the use of a solubilizer, by reducing the precipitation of thymol, can be assumed to help preventing its evaporation.

In a preferred embodiment, the alcohol-containing liquid extracts can be diluted prior to the alcohol removal in order to increase the recovery rate of volatile compounds (such as thymol or primulic acid) and to increase the removed amount of alcohol (such as ethanol).

The alcohol-containing liquid extracts can preferably be diluted to reach:
- an alcohol content about 40% or less for thyme liquid extract, and
- an alcohol content of about 20% or less for primula root liquid extract,
the percentages being expressed in volume, relative to the total volume of the alcohol-containing liquid extract.

Preferably, the removal of alcohol is operated by nanofiltration in the context of the present invention.

By using the nanofiltration method disclosed above, it was for example possible to reduce the ethanol content of the original primula root liquid extract from 73% (v/v) to 2.54% (v/v) and the ethanol content of the original thyme liquid extract from 32% (v/v) to 2.43% (v/v).

### Method for preparing an alcohol-free pharmaceutical composition through granulation

In another aspect, the invention is directed to another method for preparing said alcohol-free pharmaceutical composition comprising the step of:
i. Spraying a granulating solution comprising an alcohol-containing thyme liquid extract and alcohol-containing primula root liquid extract on a sugar matrix to obtain thyme- and primula root-extract containing granules, and
ii. Dissolving the obtained granules into a non-alcoholic solvent and excipients.

In a preferred embodiment, the granulating solution further comprises a binding agent to help granulation.

Step i. is preferably operated under heating, for example at a temperature ranging from 35 to 70°C, in order to remove the alcohol (ethanol) from the alcohol-containing liquid extract during the granules preparation.

Step i. can suitably be operated in a heated fluid bed dryer.

In one aspect of the invention, step i. of the method for preparing alcohol-free pharmaceutical compositions comprises:
- Weighting and sieving the sugar (or sucrose) filler matrix,
- Preparing a granulating solution comprising an alcohol-containing thyme liquid extract, alcohol-containing primula root liquid extract and optionally a binding agent,
- Filling the sucrose filler matrix into a heated fluid bed dryer and
- Spraying the alcohol-containing granulating solution in the heated fluid bed dryer to obtain alcohol-free thyme- and primula root-extract containing granules.

In a further preferred embodiment, step i. is followed by a drying step and a sieving step to obtain the alcohol-free thyme- and primula root-extract containing granules having a rather uniform size distribution.

In a specific embodiment of the invention, the granules were prepared in step i. using the following process:
- Preparing a granulating solution by:
   o filling thyme and primula root liquid extracts into an appropriate vessel,
   o Adding a binder such Povidone or PEG20000 while stirring,
   o Maintaining stirring for about 15-20 min at a speed ranging from 200-400 rpm (depending on stirring unit),
   o Maintaining a 50-100 rpm stirring during the whole process.
- Filling of Sucrose into the Fluid bed dryer
- Heating of Fluid bed dryer such that:
   o The temperature of the Air Supply is about 55°C,
   o The maximum Product temperature is about 40°C
   o The Volume flow rate is ranging from 1700-3000 m³/h
   o The Vibration time is about 10-20 sec
   o The Rest period vibration time is 100-120 sec
- Spraying the granulating solution under the following conditions:
   o The temperature of the Air Supply is about 55°C,
   o The maximum Product temperature is about 40°C
   o The maximum temperature air Exhaust 60°C
   o The Volume flow rate is ranging from 1800-3000 m³/h
   o The setting of the pump is fixed depending on used pump, but generally about 0.7 litre/min,
   o The Spray pressure is about 2.0-4.5 bar for a pump setting of about 0.7 litre/min
   o The Vibration time is about 20-30 sec
   o The Rest period vibration time is 60-120 sec
- Drying
   o The temperature of the Air Supply is about 55°C,
   o The maximum Product temperature is about 40°C
   o The maximum temperature air Exhaust 60°C
   o The Volume flow rate is ranging from 1800-3000 m³/h
   o The Vibration time is about 20 sec
   o The Rest period vibration time is 100-120 sec

A loss of weight if about 0.6 to 0.8% is expected during the drying step.
- Sieving of the obtained granules at a Mesh Size of about 0.8mm.

In some cases, the obtained alcohol-free thyme- and primula root-extract containing granules can be difficult to dissolve in some non-alcoholic solvents and excipients such as sucrose and/or invert sugar, and have therefore to be pre-dissolved in water, with the help of a solubilizer.

Therefore, in a preferred embodiment of the above-disclosed method for preparing an alcohol-free pharmaceutical composition through granulation, step ii. is conducted by pre-dispersing the granules obtained in step i. in water in the presence of a solubilizer, then admixing the non-alcoholic solvent and excipients.

The present invention will be further detailed in the following non limiting examples.

### Examples

### Example 1: Nanofiltration of primula root liquid extract

Primula root liquid extract (R&R Extrakte GmbH, Cologne Germany) comprising 73% (v/v) of ethanol was filtered with the help of a LabCell PF 1® unit equipped with a SelRO MPF 34® membrane. The temperature was maintained around 20°C and the transmembrane pressure was about 40 bars

The presence of primulic acid, which is a registered marker substance of the prior art product Bronchicum Elixir, in the permeate (portion of the feed that passes through the membrane) was controlled as primulic acid is expected to remain in the retentate (portion of the feed solution retained on the high pressure side of the membrane) of the filtration.

Therefore, permeate and retentate of the filtration were analysed for:
- Assay ethanol
- Assay primulic acid.

A first nanofiltration was conducted on the undiluted primula root liquid extract

The analytical results indicated that primulic acid pass the membrane in low concentrations.

In order to avoid the break-through of primulic acid, different dilutions in water of primula root liquid extract were prepared and filtered with the help of a SelRo MPF 34® membrane.

Dilution:
Sample 1: 73% Alc. V/V ► diluted to 10% V/V (analytical results see permeate 1a)
Sample 2: 73% Alc. V/V ► diluted to 25% V/V (analytical results see permeate 2a)
Sample 3: 73% Alc. V/V ► diluted to 40% V/V (analytical results see permeate 3a)
Sample 4: 73% Alc. V/V ► diluted to 55% V/V (analytical results see permeate 4a)
Sample 5: 73% Alc. V/V ► undiluted (analytical results see permeate 5a)

The following table shows the result of these experiments:

| | Primulic acid [mg/100ml] | Ethanol [% V/V] | Ethanol [% V/V] of the original sample |
|---|---|---|---|
| Permeate 1a | < 0.06* | 5.46 | 10 |
| Permeate 2a | < 0.06* | 23.45 | 25 |
| Permeate 3a | < 0.06* | 33.67 | 40 |
| Permeate 4a | < 0.17** | 50.24 | 55 |
| Permeate 5a | < 0.17** | 68.58 | 73 |

| | | | |
|---|---|---|---|
| *Detection limit of primulic acid: 0.06 mg/100ml **Quantification limit of primulic acid: 0.17 mg/100ml | | | |

In permeates 3a, 2a and 1a, there is no primulic acid detectable and the amount of ethanol, in the retentate significantly decreased

Based on these results, the diluted primula root liquid extract of sample 2 (ethanol 25% V/V), was further filtered using 6 dilution steps in order to obtain a retentate with an alcohol content of below 0,5% .

Dilution steps:
25.0% V/V►12.50%►6.25%►3.125%►1.625%►0.781%►0.391%

The following table shows the analytical result of the permeates and retentate:

| | Primulic acid [mg/100ml] | Ethanol [% V/V] | Ethanol [% V/V] of the original sample |
|---|---|---|---|
| Permeate 1b | < 0.06* | 9.43 | 12.500 |
| Permeate 2b | < 0.06* | 5.66 | 6.250 |
| Permeate 3b | < 0.06* | 3.07 | 3.125 |
| Permeate 4b | < 0.06* | 2.16 | 1.625 |
| Permeate 5b | < 0.06* | 1.31 | 0.781 |
| Permeate 6b | < 0.06* | 0.98 | 0.391 |
| Retentate b | 0.524% | 2.54 | 0.391 |
| | About 81% of original extract | | |

| | | | |
|---|---|---|---|
| *Detection limit of primulic acid: 0.06 mg/100ml | | | |

The results show that the ethanol content of the original extract was reduced from 73% V/V to 2.54% V/V.

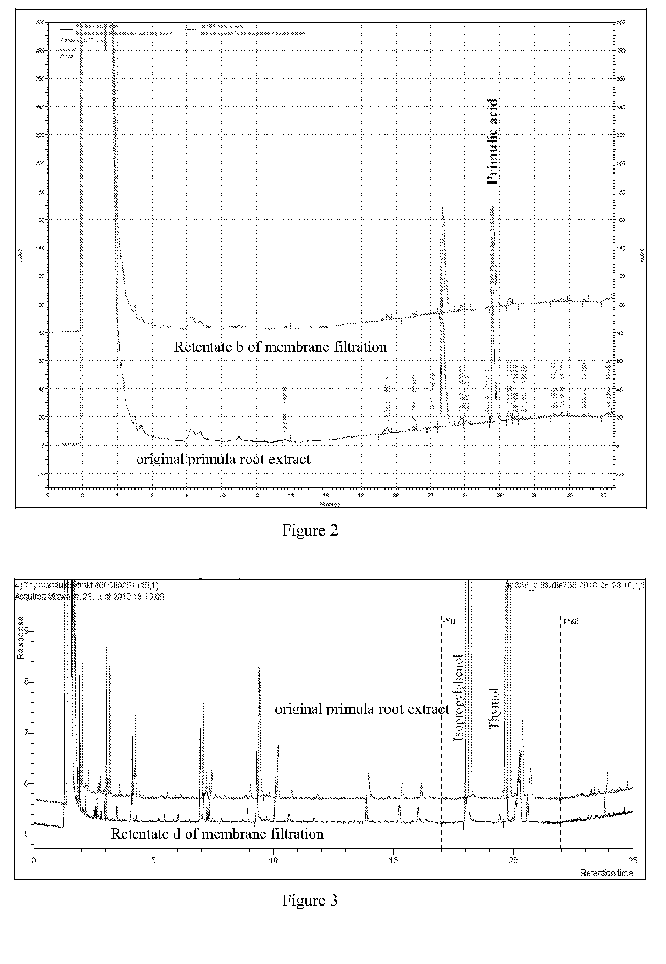

Primulic acid and other compounds did not pass the membrane. The fingerprint of the final retentate is comparable to the original extract, as can be seen from figure 2. Indeed, figure 2 illustrates the HPLC fingerprint of original primula root extract and retentate (b) of membrane filtration. The following HPLC conditions were used:

| | |
|---|---|
| Column: | Luna C18 [2], 3µm, 150 x 4,6mm |
| Flow: | 0,6 ml/min |
| Temperature: | 35 °C |
| Wavelength: | 195 nm |
| Injection: | 20 µl |
| Mobile Phase: | A: KH₂PO₄-Buffer 20mM/ Acetonitrile = 950/ 50 (V/V) |
| | B: Acetonitrile |

Gradient-program:

| Time [min] | %A | %B | Flow [ml/min] |
|---|---|---|---|
| 0 | 73 | 27 | 0.6 |
| 10 | 73 | 27 | 0.6 |
| 25 | 60 | 40 | 0.6 |
| 30 | 60 | 40 | 0.6 |
| 31 | 30 | 70 | 1.0 |
| 39 | 30 | 70 | 1.0 |
| 40 | 73 | 27 | 0.6 |
| 47 | 73 | 27 | 0.6 |

### Example 2: Filtration of primula root liquid extract with reverse osmosis

Primula root liquid extract (R&R Extrakte GmbH, Cologne Germany) comprising 73% (v/v) of ethanol was filtered using TFC®HR reverse osmosis membrane. The temperature was maintained around 20°C and the transmembrane pressure was about 40 bars.

Permeate and retentate of the filtration were here again analysed for:
- Assay ethanol
- Assay primulic acid.

The test was conducted on the undiluted primula root liquid extract

The analytical results indicated that 45.55% primulic acid did not pass the membrane and 42.96% ethanol was recovered in the permeate.

The recovery rate of primulic acid as well as the ethanol content in the permeate can easily be improved by using different dilutions of the extract.

### Example 3: Nanofiltration of thyme liquid extract

Thyme liquid extract (R&R Extrakte GmbH, Cologne Germany) comprising 32% (v/v) of ethanol was filtered with the help of a LabCell PF 1® unit equipped with a SelRO MPF 34® membrane. The temperature was maintained around 20°C and the transmembrane pressure was about 40 bars.

The presence of thymol, which is a registered marker substance of the prior art product Bronchicum Elixir, in the permeate was controlled.

Therefore, permeate and retentate of the filtration were analysed for:
- Assay ethanol
- Assay thymol.

In order to decrease the ethanol concentration, different dilutions in water of thyme liquid extract were prepared and filtered with the help of a SelRo MPF 34® membrane.

Dilution:
Sample 1: 32% Alc. V/V ► diluted to 10% V/V (analytical results see permeate 1c)
Sample 2: 32% Alc. V/V ► diluted to 15% V/V (analytical results see permeate 2c)
Sample 3: 32% Alc. V/V ► diluted to 20% V/V (analytical results see permeate 3c)
Sample 4: 32% Alc. V/V ► diluted to 25% V/V (analytical results see permeate 4c)
Sample 5: 32% Alc. V/V ► undiluted (analytical results see permeate 5c)

The following table shows the result of these experiments:

| | Thymol [mg/100ml] | Ethanol [% V/V] | Ethanol [% V/V] of the original sample |
|---|---|---|---|
| Permeate 1c | < 0.003* | 7.5 | 10 |
| Permeate 2c | < 0.003 * | 12.4 | 15 |
| Permeate 3c | 0.1 | 17.9 | 20 |
| Permeate 4c | 0.6 | 23.3 | 25 |
| Permeate 5c | 2.6 | 28.1 | 32 |

| | | | |
|---|---|---|---|
| *Detection limit of thymol: 0.003 mg/100ml Quantification limit of thymol: 0.009 mg/100ml | | | |

In permeates 2c and 1c, there is no thymol detectable and the amount of ethanol, in the retentate significantly decreased

Based on these results, the diluted thyme liquid extract of sample 2 (ethanol 15% V/V), was further filtered using 5 dilution steps in order to obtain a retentate with an alcohol content of below 0,5%.

Dilution steps:
15.0% V/V ►7.50%►3.75%►1.875%►0.938%►0.469%

The following table shows the analytical result of the permeates and retentate:

| | Thymol [mg/100ml] | Ethanol [% V/V] | Ethanol [% V/V] of the original sample |
|---|---|---|---|
| Permeate 1d | < 0.003* | 3.72 | 7.500 |
| Permeate 2d | < 0.003* | 2.54 | 3.750 |
| Permeate 3d | < 0.003* | 3.54 | 1.875 |
| Permeate 4d | 1 | 2.14 | 0.938 |
| Permeate 5d | < 0.003* | 0.96 | 0.469 |
| Retentate d | 0.0182% About 46% of original extract | 2.43 | 0.469 |

| | | | |
|---|---|---|---|
| *Detection limit of thymol: 0.003mg/100ml | | | |

The results show that the ethanol content of the original extract was reduced from 32% V/V to 2.43% V/V.

Thymol and other compounds did not pass the membrane.

However, the poor recovery rate of thymol in the retentate could be explained by the high volatility of this compound, which could have evaporated during the process and have passed off the process during the decompression of the filter unit. Such evaporation can easily be prevented by using another filter unit such as a Spirapro®.

The fingerprint of the final retentate is comparable to the original extract, as can be seen from figure 3. Indeed, figure 3 illustrates the GC fingerprint of original thyme extract and retentate (d) of membrane filtration. The following GC conditions were used:

| | |
|---|---|
| Column: | CP Sil 19 CB 50m x 0,32mm, film thickness 0.2µm |
| Carrier gas: | Hydrogen/ 70kPa |
| Combustion gas: | Hydrogen/ 120kPa air/ 120kPa |
| Make-up gas: | Nitrogen/ 130kPa |
| Injection temp.: | 275°C |
| Injection: | 1 µl |
| Detector/ temp: | FID/ 275°C |
| Temperature program: | 80°C hold 2min, to 120°C with 2°C/min, to 250°C with 20°C/min |

### Example 4: Filtration of thyme liquid extract with reverse osmosis

Thyme liquid extract (R&R Extrakte GmbH, Cologne Germany) comprising 32% (v/v) of ethanol was filtered using TFC®HR reverse osmosis membrane. The temperature was maintained around 20°C and the transmembrane pressure was about 40 bars.

Permeate and retentate of the filtration were here again analysed for:
- Assay ethanol
- Assay thymol.

The test was conducted on the undiluted thyme liquid extract

The analytical results indicated that 51.19% thymol did not pass the membrane and 24.27% ethanol was recovered in the permeate.

The recovery rate of thymol as well as the ethanol content in the permeate can be improved by using different dilutions of the extract.

### Example 5: Nanofiltration of a mixture of thyme and primula root liquid extract

A mixture of primula root (R&R Extrakte GmbH, Cologne Germany) and thyme (R&R Extrakte GmbH, Cologne Germany) liquid extracts comprising 45.67% (v/v) of ethanol and 10% of Cremophor RH 40 solubilizer was filtered with the help of a LabCell PF 1® unit equipped with a SelRO MPF 34® membrane.

Permeate and retentate of the filtration were analysed for:
- Assay ethanol
- Assay primulic acid
- Thymol.

A diluted mixture of primula root and thyme liquid extracts (ethanol 15% V/V), was further filtered using 5 dilution steps in order to obtain a retentate with an alcohol content of below 0,5%.

Dilution steps:
15.0% V/V ►7.50%►3.75%►1.875%►0.938%►0.469%

The following table shows the analytical result of the permeates and retentate:

| | Thymol [mg/100ml] | Primulic acid [mg/100ml] | Ethanol [% V/V] | Ethanol [% V/V] of the original sample |
|---|---|---|---|---|
| Permeate 1e | < 0.003* | < 0.06** | 3,54 | 7.500 |
| Permeate 2e | < 0.003* | < 0.06** | 2,08 | 3.750 |
| Permeate 3e | < 0.003* | < 0.06** | 1,57 | 1.875 |
| Permeate 4e | < 0.003* | < 0.06** | 1,17 | 0.938 |
| Permeate 5e | < 0.003* | < 0.06** | 1,00 | 0.469 |
| Retentate e | 24.7 mg/100mL About 89% of original extract | 0.842% About 96% of original extract | 2.71 | 0.469 |

| | | | | |
|---|---|---|---|---|
| *Detection limit of thymol: 0.003mg/100ml **Detection limit ofprimulic acid: 0.06 mg/100ml | | | | |

The results show that the ethanol content of the original extract was reduced from 45.67% V/V to 2.71% V/V.

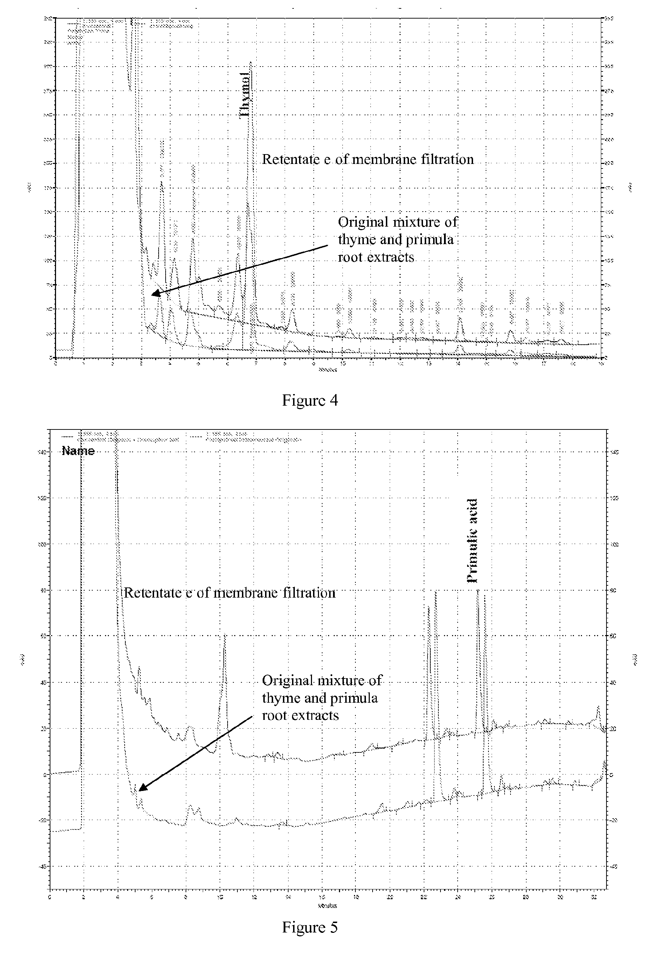

Primulic acid, thymol and other compounds did not pass the membrane. The fingerprint of the final retentate is comparable to the original mixture, as can be seen from figures 4 and 5. Indeed, figures 4 and 5 illustrate the HPLC thymol fingerprint of original mixture of primula root and thyme extract and retentate (e) of membrane filtration. Following HPLC conditions were used for the chromatogram of figure 4:

| | |
|---|---|
| Column: | Luna C18, 5µm, 150 x 4,6mm |
| Flow: | 1,0 ml/min |
| Temperature: | 35 °C |
| Wavelength: | 277 nm |
| Injection: | 10 µl |
| Mobile Phase: | A: Water |
| | B: Methanol |

Gradient-program:

| Time | %A | %B |
|---|---|---|
| [min] | | |
| 0 | 35 | 65 |
| 2 | 35 | 65 |
| 15 | 15 | 85 |
| 17 | 35 | 65 |
| 19 | 35 | 65 |

The recovery rate of thymol in the retentate (e) was 89 %.

It can be assumed that the use of a solubilizer reduces the precipitation and evaporation ofthymol.

Following HPLC conditions were used for the chromatogram of figure 5:

| | |
|---|---|
| Column: | Luna C18 [2], 3µm, 150 x 4,6mm |
| Flow: | 0,6 ml/min |
| Temperature: | 35 °C |
| Wavelength: | 195 nm |
| Injection: | 20 µl |
| Mobile Phase: | A: KH₂PO₄-Buffer 20mM/ Acetonitrile = 950/ 50 (V/V) |
| | B: Acetonitrile |

Gradient-program:

| Time [min] | %A | %B | Flow [ml/min] |
|---|---|---|---|
| 0 | 73 | 27 | 0.6 |
| 10 | 73 | 27 | 0.6 |
| 25 | 60 | 40 | 0.6 |
| 30 | 60 | 40 | 0.6 |
| 31 | 30 | 70 | 1.0 |
| 39 | 30 | 70 | 1.0 |
| 40 | 73 | 27 | 0.6 |
| 47 | 73 | 27 | 0.6 |

### Example 6: Filtration of a mixture of thyme and primula root liquid extract with reverse osmosis

A mixture of primula root (R&R Extrakte GmbH, Cologne Germany) and thyme (R&R Extrakte GmbH, Cologne Germany) liquid extracts comprising 45.67% (v/v) of ethanol and 10% of Cremophor RH 40 solubilizer was filtered using TFC®HR reverse osmosis membrane. The temperature was maintained around 20°C and the transmembrane pressure was about 40 bars.

Permeate and retentate of the filtration were here again analysed for:
- Assay ethanol
- Assay primulic acid,
- Thymol.

The test was conducted on the undiluted mixture of primula root and thyme liquid extracts.

The analytical results are as follow:

| | Thymol | Primulic Acid | Ethanol [%V/V] |
|---|---|---|---|
| Permeate | 0.25 mg/100mL | 0.34 mg/100mL | 38.66 |
| Retentate | 48.86 mg/100mL About 85% of target value | 1.35% About 86.5% of target value | 27.42 |

These results indicate that 86.5% primulic acid and 85% thymol did not pass the membrane and 38.66% ethanol was recovered in the permeate.

The recovery rate of primulic acid and thymol as well as the ethanol content in the permeate can easily be improved by using different dilutions of the extract.

### Example 7: Preparation of alcohol-free pharmaceutical composition comprising thyme and primula root liquid extracts

Two pharmaceutical compositions were prepared: formulation 1 using the single filtered extracts (examples 1 and 3) and formulation 2 using the filtered mixture of both extracts (example 7).

| Raw Materials | Function | **Formulation 1** [per 100 g] | **Formulation 2** [per 100 g] |
|---|---|---|---|
| Alcohol-free thyme liquid extract of example 1 (1 : 2-2.5) | Drug substance | 5.00 | |
| Alcohol-free primula root liquid extract of example 3 (1 : 2-2.5) | Drug substance | 2.50 | |
| Alcohol-free mixture of thyme and primula root liquid extract of example 7 | Drug substance | | 7.50 |
| Sodium benzoate | Preservative | 0.18 | 0.18 |
| Cremophor RH 40 | Solubilizer | | X |
| Mixture of sucrose and invert syrup | Flavour, Solvent | 85.00 | 85.00 |
| Purified water | Solvent | 7.32 | 7.32 |

The TLC-fingerprints indicate that the prepared pharmaceutical compositions are comparable to the original alcohol containing Bronchicum Elixir with regard to the "herbal fingerprint". The registered marker substances (thymol, primulic acid) could be detected in the finished product. Other compounds are also detectable.

### Example 8: Preparation of alcohol-free thyme- and primula root-extract containing granules

A granulating solution comprising an alcohol-containing thyme liquid extract, an alcohol-containing primula root liquid extract and povidone was sprayed on a sugar matrix.

The granules were prepared using the following process parameters:
- Preparing a granulating solution by:
   o filling thyme and primula root liquid extracts into an appropriate vessel,
   o Adding a binder such Povidone while stirring,
   o Maintaining stirring for about 15-20 min at a speed ranging from 200-400 rpm (depending on stirring unit),
   o Maintaining a 50-100 rpm stirring during the whole process.
- Filling of sucrose into the fluid bed dryer
- Heating of fluid bed dryer such that:
   o The temperature of the Air Supply is about 55°C,
   o The maximum Product temperature is about 40°C
   o The Volume flow rate is ranging from 1700-3000 m³/h
   o The Vibration time is about 10-20 sec
   o The Rest period vibration time is 100-120 sec
- Spraying the granulating solution under the following conditions:
   o The temperature of the Air Supply is about 55°C,
   o The maximum Product temperature is about 40°C
   o The maximum temperature air Exhaust 60°C
   o The Volume flow rate is ranging from 1800-3000 m³/h
   o The setting of the pump is fixed depending on used pump, but generally about 0.7 litre/min,
   o The Spray pressure is about 2.0-4.5 bar for a pump setting of about 0.7 litre/min
   o The Vibration time is about 20-30 sec
   o The Rest period vibration time is 60-120 sec
- Drying
   o The temperature of the Air Supply is about 55°C,
   o The maximum Product temperature is about 40°C
   o The maximum temperature air Exhaust 60°C
   o The Volume flow rate is ranging from 1800-3000 m³/h
   o The Vibration time is about 20 sec
   o The Rest period vibration time is 100-120 sec

A loss of weight if about 0.6 to 0.8% is expected during the drying step.
- Sieving of the obtained granules at a Mesh Size of about 0.8mm.

The resulting thyme- and primula root-extract containing granules had the following composition:

| | Granule composition [kg] |
|---|---|
| Thyme liquid extract | 0.660 |
| Primula root liquid extract | 0.330 |
| Povidone | 0.112 |
| Sucrose | 3.000 |
| Total | 4.102 |

The results showed that the herbal fingerprint of the volatile compounds of the granules is comparable to the one of the original extracts mixture.

### Example 9: Preparation of an alcohol-free pharmaceutical composition comprising thyme and primula root liquid extracts

Two pharmaceutical compositions were prepared by dissolving the granules of example 8 into non-alcoholic solvents and excipients.

In a first step, 19.87grams of granules were pre-dissolved in 20 grams of water. The resulting pre-dispersion was then admixed to other non-alcoholic solvents and additives.

The resulting alcohol-free formulation had the following composition:

| Raw Materials | **Formulation 3** [per 100 g] | **Formulation 4** [per 100 g] |
|---|---|---|
| Sodium benzoate | 0.18 | 0.18 |
| Granules of example 8 | 19.87 | 19.87 |
| Mixture of sucrose and invert syrup | 49.95 | 59.95 |
| Purified water | 30.00 | 20.00 |

The TLC-fingerprints indicate that the prepared pharmaceutical compositions are comparable to the original alcohol containing Bronchicum Elixir with regard to the "herbal fingerprint". The registered marker substances (thymol, primulic acid) could be detected in the finished product. Other compounds are also detectable.

## Claims

1. An alcohol-free liquid pharmaceutical composition comprising thyme liquid extract and primula root liquid extract and optionally a non-alcoholic solvent and excipients, preferably chosen from sucrose, invert sugar, water, and mixtures thereof.

2. The alcohol-free pharmaceutical composition according to claim 1, wherein the weight ratio of thyme liquid extract to primula root liquid extract is ranging from 2:1 to 5:1, and preferably is about 4:1.

3. The alcohol-free pharmaceutical composition according to anyone of claims 1 or 2, wherein the total amount of thyme and primula root liquid extracts is ranging from 0.1 to 10% by weight, preferably from 1 to 7% by weight, more preferably from 2 to 6% and even more preferably about 4%by weight, relative to the total weight of the formulation.

4. The alcohol-free pharmaceutical composition according to anyone of claims 1 to 3, further comprising a solubilizer, preferably chosen from triglycerides, glycerol esters, and mixtures thereof.

5. The alcohol-free pharmaceutical composition according to claim 4, wherein the solubilizer is PEG-40 hydrogenated castor oil.

6. A method for preparing an alcohol-free pharmaceutical composition according to any of claims 1 to 5 comprising the steps of:
- removing alcohol from alcohol-containing thyme and primula root liquid extracts by either nanofiltration or reversed osmosis, and,
- optionally, adding anon-alcoholic solvent and excipients, preferably chosen from sucrose, invert sugar, water, and mixtures thereof.

7. The method according to claim 6, wherein the step of removing alcohol from alcohol-containing thyme and primula root liquid extracts is performed by:
i. Removing separately the alcohol from the alcohol-containing thyme liquid extract, and from the alcohol-containing primula root liquid extract, optionally in presence of a solubilizer, by nanofiltration or reversed osmosis, or
ii. Admixing an alcohol-containing thyme liquid extract and an alcohol-containing primula root liquid extract, optionally in presence of a solubilizer, and Removing the alcohol from the alcohol-containing mixture of thyme and primula root liquid extracts by nanofiltration or reversed osmosis.

8. The method according to anyone of claims 6 or 7, wherein the alcohol-containing liquid extracts are diluted prior to the alcohol removal to reach an alcohol content of about 40% or less for thyme liquid extract, and an alcohol content of about 20% or less for primula root liquid extract, the percentages being expressed in volume, relative to the total volume of the alcohol-containing liquid extract.

9. A method for preparing an alcohol-free pharmaceutical composition according to anyone of claims 1 to 5 comprising the steps of:
i. Spraying a granulating solution comprising an alcohol-containing thyme liquid extract and alcohol-containing primula root liquid extract on a sugar matrix to obtain thyme- and primula root-extract containing granules, and
ii. Dissolving the obtained granules into a non-alcoholic solvent and excipients.

10. The method according to claim 9, wherein the granulating solution further comprises a binding agent.

11. The method according to anyone of claims 9 or 10, wherein step i. is operated under heating, at a temperature ranging from 35 to 70°C, for example in a heated fluid bed dryer, in order to remove the alcohol from the alcohol-containing liquid extract.

12. The method according to anyone of claims 9 to 11, wherein step i. is followed by a drying step and a sieving step to obtain the thyme- and primula root-extract containing granules.

13. The method according to any of claims 9 to 12, wherein step ii. is conducted by pre-dispersing the granules obtained in step i. in water in the presence of a solubilizer, then admixing the non-alcoholic solvent and excipients.

14. The method according to any of claims 6 to 13, wherein the alcohol removed from the thyme and primula root liquid extracts is ethanol.

15. A Thyme and Primula Root liquid extracts that may be obtained according to the method described in one of claims 6 to 13.

16. The use of Thyme and Primula Root liquid extract as defined in claim 15, for the preparation of a pharmaceutical composition.
